# EUROPEAN PATENT SPECIFICATION

(11) **EP 0 642 823 B1**
(45) Date of publication and mention of the grant of the patent: **26.07.2000**
(21) Application number: 94202381.3
(22) Date of filing: 22.08.1994
(51) Int. Cl.: B01D 61/14, C07K 5/06, B01D 61/18, B01D 61/02

(54) **Process for the recovery of raw materials in the aspartame preparation process**
Verfahren zur Rückgewinnung von Rohstoffen bei der Herstellung von Aspartam
Procédé pour la récupération de matériaux bruts pendant la fabrication de l'aspartam

(30) Priority: 30.08.1993 BE 9300889
(43) Date of publication of application: 15.03.1995
(73) Proprietor: HOLLAND SWEETENER COMPANY V.O.F., 6212 XW Maastricht (NL)
(72) Inventor: Meindersma, Geert Wytze, NL-6181 AD Stein (NL); Vergossen, Franciscus Hendrik Paul, NL-6104 AR Echt (NL)

(56) References cited:
- EP-A- 0 137 877
- EP-A- 0 248 416
- EP-A- 0 476 875
- WO-A-91/18666
- WO-A-92/14539
- CHEMICAL ABSTRACTS, vol. 108, no. 7, 15 February 1988, Columbus, Ohio, US; abstract no. 56620t, Y MIYAKI ET AL. 'a method for the purification of aspartame by dialysis' page 780 ; & JP-A-62 153 298 (TOYO SODA MFG CO LTD)

## Description

The invention relates to a process for the recovery of raw materials occurring in processes for the preparation of aspartame or other dipeptide sweeteners by use of a nanofiltration membrane process from aqueous process streams also containing dissolved salt.

Aspartame, the α-dipeptide ester L-aspartyl-L-phenylalanine methyl ester ("APM"), is an important synthetic low-calorie sweetening agent which is about 200 x as sweet as sugar and has an exceptionally good taste pattern without, for instance, a bitter aftertaste. The sweetener is used as such in a wide range of products such as soft drinks, sweets, table-top sweeteners, pharmaceutical products and the like. Aspartame can be prepared by various known routes. There exist, for instance, routes whereby (N-protected) L-aspartic acid or the anhydride thereof and (L-)phenylalanine or the methyl ester thereof are chemically coupled, the protecting group optionally being removed later and APM being obtained by esterification. Examples of such processes are disclosed in, for instance, US-A-3,786,039. There also exist enzymatic processes whereby, for instance, N-protected L-aspartic acid and (DL-)phenylalanine methyl ester are selectively coupled to form the LL-dipeptide derivative and subsequently converted to APM. Such a process is described in, for instance, US-A-4,116,768.

Accordingly, as starting materials in aspartame preparation processes use is often made of raw materials such as L-aspartic acid ("Asp"), L-aspartic anhydride ("AspAnh"), N-protected derivatives thereof with protecting groups such as formyl ("F") and benzyloxycarbonyl ("Z"), for instance F-AspAnh and Z-Asp, L- or DL-phenylalanine ("Phe") and the methyl ester thereof ("PM"). These starting materials are not all fully converted in the various aspartame preparation processes or are formed again through decomposition in downstream steps in the preparation in addition to other decomposition products such as 3-benzyl-6-carboxymethyl-2,5-diketopiperazine ("DKP") or L-aspartyl-L-phenylalanine ("AP") and in addition to any undesired by-products such as the β-form of APM (β-APM) and products in which the free carboxyl group of the aspartyl part of the dipeptide or the dipeptide ester has esterified, which are also referred to as AP(M) and APM₂. As a rule, for reasons of process economy, aspartame processes involve a large number of recirculation streams.
During the preparation of aspartame, especially in the working-up and recovery thereof, the process streams, including the streams involved in the preparation of raw materials or intermediate products for the aspartame process, go through various pH values as a result of, among other things, the addition of acids and bases for, for instance, the removal of protecting groups, purification, precipitation of, for instance, the Z-APM.PM addition product or the APM.HCl salt or any other addition product or crystallizing salt, and recovery of APM by crystallization at a pH of 4.0 to 5.5. These operations result in various, usually aqueous, process streams which, besides quantities of one or more of the starting materials and/or decomposition products mentioned, contain inorganic salt or inorganic salts. The aqueous streams may also contain minor amounts of organic solvents. Sodium chloride, for instance, is often present in such streams in amounts above 1% by weight, and very often in amounts as large as from 10 to 25 wt.%. Some streams from enzymatic processes may in addition contain small amounts of enzyme.

EP-A-0,476,875 discloses methods for purification and concentration of biologically active materials from admixtures containing organic solvents using solvent-stable membranes. It shows membrane processes which, using recent terminology, may be considered to be nanofiltration membrane processes (though such term is not used in the specification, and as such does not relate to processes for separation of low molecular weight organic compounds from salts). Although this document also shows examples of concentrating aqueous APM solutions (of very low concentration), there is no teaching nor suggestion that such process could be used favorably for treating such, or even higher concentrated APM solutions, which would contain amounts of salt much higher than 2000 ppm. The document in fact does not show an effective method for treatment of aspartame solutions in the presence of high amounts of salt. The membranes shown appear to have such high retention for salts (about 80% as calculated from the Examples) that they do not seem fit for lowering the amount of salt in the retentate. Moreover it should be noticed that, as can be seen from the examples on purification of penicillin G, the performance of the solvent-stable membranes does not vary over a broad range of pH values (from 0.5 to 12), but sharply deteriorates at even higher pH.

Other membrane processes in relation to the aspartame process can be mentioned as well. EP-A-0,313,100, for instance, describes an electrodialysis process by which in particular organic acids such as DKP and AP can be removed. In JP-A-62-153,298 it is indicated that APM can be cleared of low-molecular weight electrolytes present in it by dissolving the APM in water and contacting the solution in a dialysis process at a pH of 3-7 and at a temperature of 0-80°C with an amphoteric ion exchanging membrane allowing the low-molecular weight electrolytes to pass, the volume of the original solution being retained. In such a process, an increase in pressure does lead to an increase in concentration, but also leads to unwanted permeation losses of organic products. Moreover, in a dialysis process the dialysate always needs to be replaced.

Also, EP-B-0,248,416 discloses a salt removal process which - at elevated pressure - is said to operate on the principle of reverse osmosis. That process utilizes neutral membranes such as polyamide acetate, polysulphone acetate and cellulose acetate membranes with 30-80% salt retention. At a lower salt retention such membranes are not suitable because too much organic material will be transported and there will be no fractionating effect with respect to molecules larger than 100D (1D = 1 Dalton is 1/16th of the mass of the oxygen-16 isotope). Moreover, in such a process the extent to which the starting solution can be concentrated is limited inasmuch as the osmotic pressure strongly increases during the process.

The raw materials still to be recovered often are present in relatively dilute streams. The presence of salt in the streams, however, makes it difficult to strongly concentrate the streams by evaporation, because when evaporation takes place in the presence of salt, the salt, given the unfavourable weight ratio of the organic components and the salt, is bound to crystallize or cocrystallize with organic components and, moreover, undesired side-reactions may take place, causing for instance strong discoloration.

None of the working-up processes described so far has been found to be generally suitable for recovering raw materials from those aqueous process streams in the aspartame preparation processes which contain high amounts of salt of at least 1% by weight. Furthermore, processes such as dialysis and electrodialysis, reverse osmosis and treatment with an ion exchanger are relatively cumbersome and less desirable on an industrial scale. Moreover, in most of the prior art methods the solution started from can be concentrated only to a limited extent. As a consequence, there is a need for a process that does not have these drawbacks and enables the raw materials under consideration to be recovered easily and efficiently in a process involving simultaneous salt removal from the said process streams and concentration of the organic components in them.

The object of the invention is to provide a process whereby raw materials are recovered easily and efficiently, with any previously present salt being to a large extent removed, from various process streams in aspartame preparation processes, the organic components in the process stream or streams concerned being concentrated simultaneously.

This object is achieved by the process of claim 1. For the purposes of the present invention, nanofiltration should be understood to mean the application of membranes possessing both reverse osmosis and fractionating properties with respect to molecules larger than 100D. This means that membranes used in the context of the invention should possess such properties as render them suitable for application in reverse osmosis and for application in fractionating molecules larger than 100D.

The per cent retention of a component i (Rᵢ) is given by the formula ${\text{R}}_{\text{i}} {\text{= (1-C}}_{\text{ip}} {\text{/C}}_{\text{ir}} \text{) x 100%}$ , wherein Cᵢₚ = the concentration of component i in the permeate and Cᵢᵣ = the concentration of component i in the retentate, both expressed in wt.%.

Preferably, use is made of composite membranes that have a substantially negatively charged selective top layer which has been applied to an ultrafiltration membrane as supporting layer.

Such membranes are commercially available. Membranes suitable for the purpose of the invention are for instance of the SelRo®-type (from Kiryat Weizmann Ltd.), DRC-1000® (from Celfa), NF-PES-10/PP60® (from Kalle) and NTR 7410® (from Nitto).
It will be clear to those skilled in the art that aspartame, as referred to in this application, should be taken to include other dipeptide sweeteners such as L-aspartyl-D-alaninyl-2,2,4,4-tetramethylthietanyl amide (alitame). Likewise, as to the raw materials to be recovered the invention is not strictly limited to such raw materials as are used in the known aspartame preparation processes but also covers those raw materials which are not used as building blocks for aspartame but which can be used for the preparation of other dipeptide sweeteners. The invention equally includes the recovery of raw materials for aspartame preparation from recirculation streams or waste streams which occur in the preparation processes for those same raw materials, such as Phe, Z-Asp, Asp, PM and the like. Where streams contain substantial amounts of APM it is recommended first to recover the APM. It will also be clear that the invention relates not only to streams also containing small amounts of dissolved APM - whether or not after removing the bulk of the APM - but also to streams devoid or almost devoid of APM. However, any APM present in the process streams will also be recovered, whether or not by way of conversion to the raw materials.
Needless to say, streams containing only very small amounts of raw materials will not be subjected to this recovery process according to the invention. Nor, as a rule, need the permeate streams that are obtained in applying the process and that contain far lower raw materials concentrations than the treated process streams be subjected to a second treatment according to the invention. If they are of acceptable quality, the retentate streams obtained may be returned to the preparation process, whether or not after adjusting of, for instance, the pH and/or temperature, but usually without any further processing, or may be utilized to recover the desired components by commonly known methods.

Those skilled in the art will be able to establish which process streams are, and which process streams are not, suitable for such a nanofiltration treatment, depending on the desired economy of the overall process.

The process according to the invention is particularly suitable for recovering Z-Asp, F-Asp, Asp, AspAnh, Phe and PM from aqueous streams from the aspartame process. The choice of a pH of 8-11 at which nanofiltration is effected may affect the composition of the streams. At a pH > 7, PM, for instance, will hydrolyse and Phe can be recovered.

The process can be operated batch-wise or continuously. This can be effected in apparatus known per se such as that employed for reverse osmosis and ultrafiltration. Such apparatus generally comprises one or more membrane modules through which the liquid is pumped under pressure by one or more pumps.

Processes for the preparation of aspartame include many streams that may be treated according to the present invention. Examples of components that may occur in such streams have already been given in the foregoing (e.g. APM, raw materials such as PM, Z-Asp, Asp, F-Asp, Phe and decomposition products or by-products such as AP, DKP, AP(M), APM₂, etc.). The concentrations in which these substances occur vary depending on the process or the process section in which the stream is present and may vary between wide limits depending on the solubility, temperature, pH and so forth.

Concentrations of 0.1-5% are common. Some streams, particularly in enzymatic processes, may also contain some enzyme.

In the process according to the invention, the process stream/streams to be treated is/are supplied individually or collectively to one side of a membrane module in which a nanofiltration membrane effects the separation between this side of the module and the other side, from where the permeate that has passed through the membrane and that has largely been cleared of organic compounds of Mw ≥ 100D can be discharged. If tubular membranes are employed, the process stream/streams to be treated is/are supplied on the inside of the tube and discharged on the outside, or the other way round. Spiral-wound, hollow-fibre, capillary or flat membranes may also be used.

Thus, on the inlet side a retentate solution is obtained in which the organic components are more concentrated and in which the ratio of the organic components and salt is much improved and from which the raw materials can be recovered by simple, commonly known methods; it is also often possible to return the retentate solution to the process. If the organic components are present in relatively high concentrations, one or more of such components may crystallize to some degree as the retentate solution is being concentrated. This has not been found to be unfavourable for the process of the invention, as will also be evident from the experimental part. The permeate flux and/or the separation efficiency may be improved still further by proper adjustment of the pH and pressure on the inlet side of the nanofiltration membrane. A pH from 8-11 is chosen and the system is operated at a pressure on the inlet side which is higher than atmospheric, preferably higher than 1 MPa. The upper limits of pH, temperature and pressure depend in part on the properties of the membrane.

The temperature at which nanofiltration is effected is generally not critical and is between 0 and 80°C depending on the membrane chosen.

Ambient temperature is particularly suitable. However, at higher temperatures a higher flux is attained because of the favourable viscosity of the streams, and this may be advantageous also.

The (proper) performance of the nanofiltration process according to the invention can be established by determining (1) the concentration factor (Cf), by which is meant the quotient of the amounts of feed and retentate, and (2) the retention for each of the desired components (Rᵢ), and (3) the permeate flux (Fp) in kg/m².h, being the amount of permeate passing through a unit area of the membrane per unit time.
After use - or when the permeability of the membrane diminishes - the membranes may be readily cleaned, at a slightly elevated temperature if so desired, by rinsing with water or a caustic solution or, if the streams treated contain small amounts of enzyme, by rinsing in the presence of a small amount of surfactant, for instance Ultrasil®.
The higher the pressure, the higher the permeate flux will be. An increase in pH, too, will result in a greater flux. Increasing the pH from 4 to 6 (NOT THE INVENTION) to pH values of approx. 8-9 leads to an increase in values found for the retention (Rᵢ) of the organic components and a decrease in the retention for salts. At pH values > 9.9 hardly any effect on the retention (Rᵢ) is observed. Increasing the pressure also leads to an increase in the retention figure for the raw materials.
The higher the value of Cf chosen (by adjusting the process conditions), the greater (in absolute terms) the portion of raw materials that will be discharged via the permeate, so that the amount of raw materials to be reclaimed from the retentate will decrease. Therefore, if more raw material is to be recovered via the retentate, a lower concentration factor should be chosen. In that case, the membrane area may be smaller than would be necessary for attaining a higher Cf.

It will be easy for those skilled in the art to determine favourable settings enabling the desired results and economy of the process to be achieved. The nature of any salt or salts present in the streams, too, has been found to affect the retention thereof. In general, the retention is lower for monovalent salts. Since the nature of the salt in the process streams is dependent on the acids and bases used in the various process steps of the aspartame preparation process, it is preferred to use monovalent acid radical ions and metal ions so that sodium chloride, for instance, is formed as salt.

It is also possible to recirculate the permeate obtained and to add it to the stream to be subjected to nanofiltration or to subject it again to a separate nanofiltration treatment. In that way, the amount of the raw materials to be recovered can be increased further. It has been found that, even if the concentration of the raw materials in the streams to be subjected to nanofiltration according to the invention becomes so high during nanofiltration that one or more of such raw materials crystallize, the performance of the nanofiltration in terms of the retention effect is not affected. However, the permeate flux will diminish as a result of crystals precipitating on the membrane surface. Additional amounts of salt can be removed while continuing the nanofiltration and without any decrease in permeate flux by adding water or a buffer solution on the inlet side just before crystallization can take place. In that case, a combination of nanofiltration and diafiltration is applied. Even when there is no question of crystallization, this method may be applied for further removal of salt from the retentate.

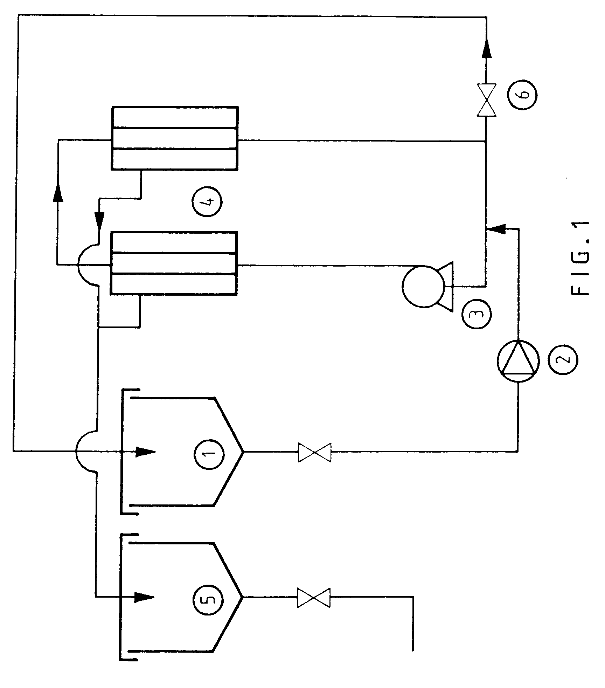

The invention is illustrated by the following non-limiting examples. All of the experiments described below were carried out in a rig as depicted in Fig. 1. In this figure:
- 1.: is a (20-litre) storage tank for the solution to be treated, equipped with a stirrer. The contents of this tank are kept at a temperature of about 40°C. The retentate formed in the experiments was always returned to this tank and mixed with its contents.
- 2.: is a feed pump (with a maximum throughput of 100 l/h) which pressurizes the liquid and keeps it under pressure in combination with valve 6.
- 3.: is a circulation pump whose throughput is adjustable, to a maximum of 1800 l/h, and which supplies the feed to the inside of the tubular membranes in
- 4.: a membrane module system with two series-arranged tubular composite membranes and separate discharge streams for permeate and retentate.
- 5.: is a collecting vessel for the permeate formed in the experiments.
- 6.: is a pressure control valve.
Prior to each of the following experiments 10-20 litres of an aqueous, salt-containing, representative process stream was prepared by adding one or more organic components, NaCl and 22% NaOH solution until the concentrations listed in Tables 1 and 3 were obtained. The concentrations of the organic components were determined with the aid of HPLC (high pressure liquid chromatography) and the chloride concentration was determined with the aid of ion chromatography. The pH was measured at the temperature of the solution.
In all nanofiltration experiments the solution started from was charged to the storage tank at 40°C, whereupon the feed pump and the circulation pump were started. During most experiments a pressure of 3 MPa (30 bar) was maintained. However, in some cases a different pressure setting was applied, as indicated.

### I. Use of SelRo MPT 10® membrane

A first set of experiments were carried out with the aid of a PCI Microlab 80 module (Fig. 1, No. 4) in which two tubular SelRo MPT 10® membranes with a diameter of 12.5 mm and a length of 1.2 m were arranged in series.
The starting solutions used in experiments 1-6 are listed in Table 1. All experiments were carried out at a pressure of 3 MPa (30 bar), 40°C and a maximum throughput of the pumps. Table 1 also gives the composition for comparative experiment A, which is described at the end of the experimental section.

**TABLE 1**

| | composition in wt.% | | | | pH |
|---|---|---|---|---|---|
| | Phe | PM | Z-Asp | Cl⁻ | |
| 1 (comp.) | 1.18 | 0.64 | 0.64 | 7.18 | 4.6 |
| 2 | 1.2 | - | 0.65 | 6.5 | 8.4 |
| 3 | 1.8 | - | 0.63 | 6.3 | 9.9 |
| 4 (comp.) | 1.44 | - | - | 10.10 | 6.1 |
| 5 | 1.35 | - | - | 9.84 | 8.1 |
| 6 | 1.02 | - | 1.02 | 11.89 | 9.8 |
| A (comp.) | 1.06 | 0.52 | 0.50 | 7.86 | 4.6 |

During the experiments no change in pH was observed. The results of the experiments in terms of permeate and retentate composition after the point where the randomly chosen concentration factor Cf (also indicated) was reached are summarized in Table 2, which also includes the values of Rᵢ, Cf and Fp for the end situation. The results of Comparative experiment A (see below) are also included.

**TABLE 2**

| | Cf | Fp | permeate (wt.%) | | | | retentate (wt.%) | | | | Rᵢ (%) | | | |
|---|---|---|---|---|---|---|---|---|---|---|---|---|---|---|
| | [-] | kg/m². hr | Phe | PM | Z-Asp | Cl⁻ | Phe | PM | Z-Asp | Cl⁻ | Phe | PM | Z-Asp | Cl⁻ |
| 1* | 4.0 | 21 | 0.73 | 0.59 | 0.36 | 7.70 | 2.80 | 1.28 | 1.94 | 7.50 | 74 | 54 | 81 | -3 |
| 2 | 5.8 | 27 | 1.2 | - | 0.24 | 6.80 | 4.40 | - | 3.01 | 5.78 | 73 | - | 92 | -18 |
| 3 | 5.1 | 49 | 1.6 | - | 0.25 | 6.60 | 4.40 | - | 2.42 | 5.50 | 64 | - | 90 | -20 |
| 4* | 3.7 | 58 | 0.9 | - | - | 10.50 | 2.63 | - | - | 9.28 | 66 | - | - | -13 |
| 5 | 4.3 | 70 | 0.89 | - | - | 10.3 | 2.14 | - | - | 9.18 | 58 | - | | -12 |
| 6 | 3.9 | 55 | 0.8 | - | 0.8 | 12.38 | 2.50 | - | 2.70 | 10.23 | 68 | - | 70 | -21 |
| A* | 1.3 | 25 | 0.31 | 0.29 | 0.06 | 5.42 | 1.29 | 0.59 | 0.64 | 8.61 | 76 | 51 | 91 | +37 |

| | | | | | | | | | | | | | | |
|---|---|---|---|---|---|---|---|---|---|---|---|---|---|---|
| *: comparative | | | | | | | | | | | | | | |

In some of these experiments (Nos. 1 and 2) Phe had crystallized in the retentate during the experiment. This did not affect the Rᵢ-values.

### II. Use of WFN 0505 membrane

In the same way as described above for SelRo MPT10® membrane, experiments were performed in a membrane module which had been slightly modified because of the different diameter and length of the membranes (Figure 1, No. 4).
Each of the WFN tubular membranes was 14.4 mm in diameter and 1.8 m long.
The solutions started from in these experiments are listed in Table 3. The results of the experiments are given in Table 4. These experiments, too, were carried out at 3 MPa (30 bar) and 40°C.

**TABLE 3**

| | Composition in wt.% | | | | pH |
|---|---|---|---|---|---|
| | Phe | PM | Z-Asp | Cl⁻ | |
| 7*) | 1.00 | 0.51 | 0.91 | 7.21 | 4.6 |
| 8**) | 1.59 | - | 0.98 | 6.40 | 9.9 |
| 9**) | 1.59 | - | 0.98 | 6.40 | 10.6 |

| | | | | | |
|---|---|---|---|---|---|
| *) comparative | | | | | |
| **) During these nanofiltration experiments the pH was kept constant by adding small amounts of 22% NaOH solution in order to avoid changes in pH. | | | | | |

**TABLE 4**

| | Cf | Fp | permeate (wt.%) | | | | retentate (wt.%) | | | | Rᵢ (%) | | | |
|---|---|---|---|---|---|---|---|---|---|---|---|---|---|---|
| | [-] | kg/m². hr | Phe | PM | Z-Asp | Cl⁻ | Phe | PM | Z-Asp | Cl⁻ | Phe | PM | Z-Asp | Cl⁻ |
| 7*) | 2.1 | 68 | 0.31 | 0.30 | 0.17 | 6.90 | 1.73 | 0.72 | 1.69 | 7.31 | 82 | 58 | 90 | |
| 8 | 2.0 | 141 | 0.54 | - | 0.04 | 6.50 | 2.45 | - | 1.80 | 6.10 | 78 | - | 98 | -7 |
| 9 | 4.5 | 50 | 0.74 | - | 0.08 | 4.40 | 4.31 | - | 4.70 | 3.16 | 83 | - | 98 | -39 |

| | | | | | | | | | | | | | | |
|---|---|---|---|---|---|---|---|---|---|---|---|---|---|---|
| *) comparative | | | | | | | | | | | | | | |

### III. Experiments involving a combination of nanofiltration and diafiltration

Experiments were carried out on the analogy of Nos. 1 and 2, except that the compositions were slightly different and that the experiments were in part carried out under different pressures, the Phe concentration being kept relatively low by additions of water of 40°C. Phe can crystallize at around 2 wt.%, depending on the composition of the solution. The amount of water added in these experiments was about 50 wt.% referred to the amount of retentate present at the time the addition was made.
The results of these experiments, which were started with the compositions listed in Table 5, are given in Table 6 for the situations before and after addition of water as well as for the end situation (a, b and c, respectively).
Experiment 10 was carried out at 3 MPa (30 bar) and 40°C; experiment 11 at 2 MPa (20 bar) and 40°C.

**TABLE 5**

| | Composition in wt.% | | | | pH |
|---|---|---|---|---|---|
| | Phe | PM | Z-Asp | Cl⁻ | |
| 10*) | 1.16 | 0.48 | 0.47 | 5.47 | 4.6 |
| 11 | 1.86 | - | 0.52 | 6.40 | 8.4 |

| | | | | | |
|---|---|---|---|---|---|
| *) comparative | | | | | |

In experiment 11 some crystallization of Phe occurred before water was added. Due to the dilution the crystals could be redissolved. In the end a Cf of 2 could still be achieved, without any crystals being present.

**TABLE 6**

| | Cf | Fp | permeate (wt.%) | | | | retentate (%) | | | | Rᵢ (%) | | | |
|---|---|---|---|---|---|---|---|---|---|---|---|---|---|---|
| | [-] | kg/m². hr | Phe | PM | Z-Asp | Cl⁻ | Phe | PM | Z-Asp | Cl⁻ | Phe | PM | Z-Asp | Cl⁻ |
| 10a*) | 1.8 | 55 | 0.32 | 0.15 | 0.19 | 4.90 | 1.77 | 0.65 | 0.75 | 5.83 | 82 | 77 | 88 | +16 |
| 10b*) | 1.3 | 57.5 | 0.34 | 0.15 | 0.09 | 4.40 | 1.32 | 0.48 | 0.53 | 4.14 | 74 | 69 | 83 | - |
| 10c*) | 1.8 | 53 | 0.27 | 0.12 | 0.01 | 3.80 | 1.72 | 0.61 | 0.72 | 4.44 | 84 | 80 | 99 | -14 |
| 11a | 2.3 | 6 | 1.01 | - | 0.12 | 6.40 | 2.75 | - | 1.08 | 5.96 | 63 | - | 89 | -7 |
| 11b | 1.7 | 30 | 0.98 | - | 0.10 | 4.80 | 2.00 | - | 0.81 | 4.31 | 51 | - | 88 | -11 |
| 11c | 2.0 | 34 | 1.27 | - | 0.08 | 4.70 | 2.19 | - | 0.95 | 4.19 | 42 | - | 92 | -12 |

| | | | | | | | | | | | | | | |
|---|---|---|---|---|---|---|---|---|---|---|---|---|---|---|
| *) comparative | | | | | | | | | | | | | | |

### Comparative example A: reverse osmosis

For the purpose of comparison an experiment was performed in which a - neutral - AFC 30 polyamide membrane from PCI was used in a reverse-osmosis experiment in a BUF unit containing 18 of these tubular membranes with two sequences of 9 series-arranged membranes being arranged in parallel, the total length being 1.2 m and the diameter 12.5 mm (total membrane surface area 0.9 m²).

The compositions at the start and at the end of the experiments are listed in Tables 1 and 2.

## Claims

1. Process for the recovery of raw materials occurring in processes for the preparation of aspartame or other dipeptide sweeteners from aqueous process streams also containing dissolved salt, wherein each of the aqueous process streams or a combination of two or more of such streams having a salt content of at least 1% by weight is subjected to nanofiltration at a pH of 8-11 and a temperature of between 0 and 80°C with the aid of a composite membrane with a retention of 50 to 100% for components having a molecular weight higher than 100D, and which gives a retention of +20 to -40% for monovalent salts under those process conditions, the raw materials present in the retentate so obtained being recovered or the retentate being returned to the aspartame or dipeptide sweetener preparation process without any further processing.

2. Process according to Claim 1, characterized in that the composite membrane contains a substantially negatively charged top layer which is applied to an ultrafiltration membrane as a support layer.

3. Process according to any one of Claims 1 or 2, characterized in that the pressure at the feed side of the membrane is superatmospheric.

4. Process according to Claim 3, characterized in that the pressure has a value of from 1-5 MPa (10 to 50 bar) abs.

5. Process according to any one of Claims 1-4, characterized in that the dissolved salt is a monovalent salt.

6. Process according to any one of Claims 1-5, characterized in that the membrane is periodically cleaned by flushing with water or a caustic solution, optionally in the presence of a small amount of surfactant.

7. Process according to any one of Claims 1-6, characterized in that in the course of the nanofiltration process water or a buffer solution is added to the retentate and nanofiltration is continued.

## Patentansprüche

1. Verfahren für die Rückgewinnung von Rohmaterialien, die in Verfahren für die Herstellung von Aspartam oder anderen Dipeptid-Süßstoffen vorkommen, aus wässerigen Verfahrensströmen welche ebenfalls gelöstes Salz enthalten, wobei jeder der wässerigen Verfahrensströme oder eine Kombination von zwei oder mehr solcher Ströme mit einem Salzgehalt von mindestens 1 Gew.-% Nanofiltration bei einem pH von 8-11 und einer Temperatur zwischen 0 und 80°C unterworfen wird, mit Hilfe einer Verbundmembran mit einer Retention von 50 bis 100% für Bestandteile mit einem höheren Molekulargewicht als 100D, welche unter jenen Verfahrensbedingungen eine Retention von +20 bis -40% für einwertige Salze ergibt, wobei die Rohmaterialien, die in dem derart erhaltenen Retentat vorhanden sind, rückgewonnen werden oder das Retentat ohne weitere Bearbeitung zu dem Aspartam- oder Dipeptid-Süßstoff-Herstellungsverfahren rückgeführt wird.

2. Verfahren gemäß Anspruch 1, dadurch gekennzeichnet, daß die Verbundmembran eine im wesentlichen negativ geladene Oberschicht enthält, welche auf einer Ultrafiltrationsmembran als Trägerschicht aufgetragen ist.

3. Verfahren gemäß einem der Ansprüche 1 oder 2, dadurch gekennzeichnet, daß der Druck an der Beschickungsseite der Membran überatmosphärisch ist.

4. Verfahren gemäß Anspruch 3, dadurch gekennzeichnet, daß der Druck einen Wert von 1-5 MPa (10 bis 50 bar) abs. hat.

5. Verfahren gemäß einem der Ansprüche 1-4, dadurch gekennzeichnet, daß das gelöste Salz ein einwertiges Salz ist.

6. Verfahren gemäß einem der Ansprüche 1-5, dadurch gekennzeichnet, daß die Membran regelmäßig durch Spülen mit Wasser oder einer kaustischen Lösung, gegebenenfalls in Anwesenheit einer geringen Mange an Surfactant, gereinigt wird.

7. Verfahren gemäß einem der Ansprüche 1-6, dadurch gekennzeichnet, daß in dem Verlauf des Nanofiltrationsverfahrens Wasser oder eine Pufferlösung zu dem Retentat zugegeben wird und Nanofiltration fortgesetzt wird.

## Revendications

1. Processus de récupération de matières premières se produisant au cours de processus de préparation de l'aspartame ou d'autres édulcorants à base de dipeptides, à partir de flux de processus aqueux contenant également un sel dissous, où chacun des flux de processus aqueux ou une combinaison de deux, ou plus, de ces flux ayant une teneur en sel d'au moins 1 % en poids est soumis à une nanofiltration, avec un pH compris entre 8 et 11 et à une température comprise entre 0°C et 80°C, à l'aide d'une membrane composite ayant une rétention comprise entre 50 % et 100 % pour des composants ayant un poids moléculaire supérieur à 100D, et qui fournit une rétention comprise entre +20 % et -40 % pour des sels monovalents dans ces conditions de processus, les matières premières présentes dans le rétentat ainsi obtenu étant récupérées, ou bien le rétentat repasse dans le processus de préparation de l'aspartame ou de l'édulcorant à base de dipeptides sans autre étape de traitement.

2. Processus selon la revendication 1, caractérisé en ce que la membrane composite contient une charge supérieure chargée pratiquement de façon négative qui est appliquée sur une membrane d'ultrafiltration servant de couche support.

3. Processus selon la revendication 1 ou 2, caractérisé en ce que la pression, au niveau du côté alimentation de la membrane, est superatmosphérique.

4. Processus selon la revendication 3, caractérisé en ce que la pression a une valeur comprise entre 1 MPa et 5 MPa (entre 10 bar et 50 bar) abs.

5. Processus selon l'une quelconque des revendications 1 à 4, caractérisé en ce que le sel dissous est un sel monovalent.

6. Processus selon l'une quelconque des revendications 1 à 5, caractérisé en ce que la membrane est périodiquement nettoyée en rinçant avec de l'eau ou avec une solution caustique, le cas échéant en présence d'une faible quantité de surfactant.

7. Processus selon l'une quelconque des revendications 1 à 6, caractérisé en ce que, au cours du processus de nanofiltration, de l'eau ou une solution tampon est ajoutée au rétentat et on continue la nanofiltration.
